# EUROPEAN PATENT APPLICATION

(11) **EP 4 600 248 A1**
(43) Date of publication of application: **13.08.2025**
(21) Application number: 23874272.0
(22) Date of filing: 07.08.2023
(51) Int. Cl.: C07D 498/20, C07F 9/6561, C07F 9/6574, A61K 31/5386, A61K 31/675, A61P 31/16

(54) **ANTI-INFLUENZA VIRUS DERIVATIVE AND USE THEREOF**

(30) Priority: 08.10.2022 CN 202211221175; 09.11.2022 CN 202211395795; 16.05.2023 CN 202310548302
(71) Applicant: SHIJIAZHUANG DISCOVERY MEDICINE TECHNOLOGY CO., LTD, Shijiazhuang, Hebei 050035 (CN)
(72) Inventor: ZHANG, Zhefeng, Shijiazhuang, Hebei 050035 (CN); LI, Haide, Shijiazhuang, Hebei 050035 (CN); HOU, Wen, Shijiazhuang, Hebei 050035 (CN); FU, Haijian, Shijiazhuang, Hebei 050035 (CN); JIANG, Long, Shijiazhuang, Hebei 050035 (CN); PAN, Wei, Shijiazhuang, Hebei 050035 (CN)
(74) Representative: Chung, Hoi Kan
(86) International application number: PCT/CN2023/111457
(87) International publication number: WO 2024/074080

(57) **Abstract**

The present application discloses a highly effective antiviral derivative and its use; the derivative is a compound as shown in the following formula (I-0) or hydrate, solvate, optical isomer, polymorph, isotopic derivative, pharmaceutically acceptable salt thereof, and a preparation method of the derivative is also disclosed. The compound of the present application can be used for the preparation of drugs for preventing/treating influenza virus.

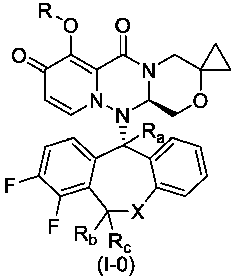

## Description

### TECHNICAL FIELD

The present invention relates to compounds having anti-influenza virus activity or its hydrates, solvates, optical isomers, polymorphs, isotopic derivatives, or pharmaceutically acceptable salts thereof, and preparation methods and uses thereof in anti-influenza virus.

### BACKGROUND

Influenza viruses mainly include four types: influenza A virus, influenza B virus, influenza C virus and influenza D virus. The main anti-influenza virus drugs on the market are amantadine, neuraminidase inhibitors oseltamivir or zanamivir. However, drug resistance has also been found in these compounds since their use.

The RNA polymerase of influenza virus contains a cap-dependent endonuclease, and inhibiting the activity of the cap-dependent endonuclease can inhibit the proliferation of the pathogenic virus. At present, this enzyme has become a promising target for the development of antiviral drugs, and many companies have turned their attention to the cap-dependent endonuclease, and different heterocyclic compounds have been used as cap-dependent endonuclease inhibitors. Even the currently marketed cap-dependent endonuclease inhibitor baloxavir, there have been reports of drug resistance, in addition, these compounds mostly show poor physicochemical properties, such as low solubility, low bioavailability, etc., so it is still necessary to develop a new generation of cap-dependent endonuclease inhibitors.

After careful research, we found that a class of seleno compounds containing cyclopropyl groups had high and broad-spectrum anti-influenza virus effects, and further research showed that the compounds had specific distribution and were expected to be developed into anti-influenza virus drugs. The compounds of the present invention inhibit viral replication by inhibiting the cap-dependent endonuclease in influenza virus, targeting an earlier stage of the viral replication cycle, thus having better effects in preventing and treating influenza.

### SUMMARY OF THE INVENTION

Unless otherwise defined herein, scientific and technical terms used in connection with the present application shall have the meanings that are commonly understood by those of ordinary skill in the art.

The present application provides a class of compounds with anti-influenza virus effect and the preparation method and use thereof.

The present application provides a compound as shown in formula (I-0), or its hydrate, solvate, optical isomer, polymorph, isotopic derivative, or pharmaceutically acceptable salt thereof, wherein: 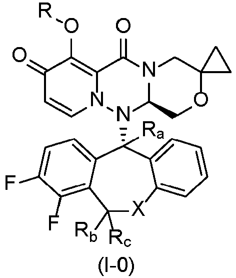
in the formula (I-0), Rₐ is selected from the group consisting of hydrogen, deuterium, methyl and deuterated methyl;
X is S or Se, and when X is S, at least one deuterium atom is contained in the formula (1-0);
R_{b} and R_{c} are each independently hydrogen, deuterium, C1-C3 alkyl, deuterated C1-C3 alkyl, or R_{b}, R_{c}, and the carbon atom to which they are attached together comprise cyclopropyl or deuterated cyclopropyl;
R is hydrogen, 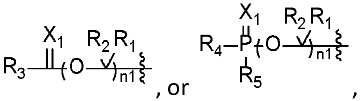 wherein:
   X1 is an oxygen atom or a sulfur atom;
   n1 is 0, 1 or 2;
   each R₁ or R₂ is independently selected from the group consisting of hydrogen, deuterium, methyl and deuterated methyl;
   R₃ is selected from a group wherein one or more hydrogen atoms are substituted or unsubstituted by deuterium, the group consisting of C1-C8 alkyl, C1-C8 alkoxy, C1-C8 alkylthio, and C1-C8 alkylamino.
   R₄ and R₅ are each independently hydroxy; or are each independently selected from the group consisting of C1-C8 alkoxy, C1-C8 alkylthio, C1-C8 alkylamino, C6-C20 aralkyloxy, one or more hydrogen atoms of each of the said groups being substituted or unsubstituted by deuterium; and R₄ and R₅ together with the phosphorus atom to which they are attached form a 5-7 membered ring of 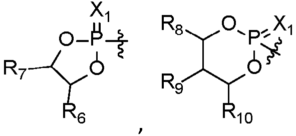 or 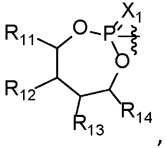 **wherein** R₆, R₇, R₈, R₉, R₁₀, R₁₁, R₁₂, R₁₃, and R₁₄ are each independently hydrogen or C1-C3 alkyl, or R₆ and R₇, R₅ and R₉, R₁₁ and R₁₂, R₁₂ and R₁₃ each together with the carbon atoms to which they are attached form an aromatic ring, wherein one or more of the hydrogen atoms in the substituent of the 5-7 membered ring formed by R₄ and R₅ together with the phosphorus atom to which they are attached can be substituted or unsubstituted by deuterium.

In some embodiments, the present application provides a compound represented by formula (I) or its hydrate, solvate, optical isomer, polymorph, isotopic derivative, or pharmaceutically acceptable salt, wherein: 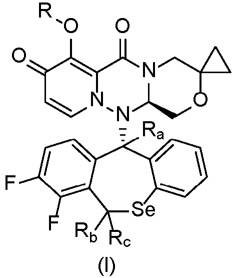
in Formula (I), Rₐ is selected from the group consisting of hydrogen, deuterium and methyl;
R_{b} and R_{c} are each independently hydrogen, deuterium, C1-C3 alkyl, or R_{b}, R_{c}, and the carbon atom to which they are attached together comprise cyclopropyl;
R is hydrogen, 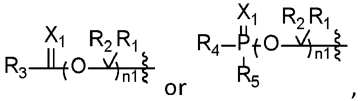 wherein:
   X₁ is an oxygen atom or a sulfur atom;
   n1 is 0, 1 or 2;
   each R₁ or R₂ is independently hydrogen or methyl;
   R₃ is selected from the group consisting of C1-C8 alkyl, C1-C8 alkoxy, C1-C8 alkylthio and C1-C8 alkylamino;
   R₄ and R₅ are each independently selected from the group consisting of hydroxy, C1-C8 alkoxy, C1-C8 alkylthio, and C1-C8 alkylamino.

In some embodiments, the present application provides a compound represented by formula (I) or its hydrate, solvate, optical isomer, polymorph, isotopic derivative, or pharmaceutically acceptable salt, wherein: 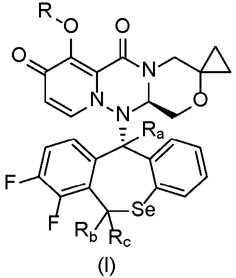
in Formula (I), Rₐ is selected from the group consisting of hydrogen, deuterium, methyl and deuterated methyl;
R_{b} and R_{c} are each independently hydrogen, deuterium, C1-C3 alkyl, deuterated C1-C3 alkyl, or R_{b}, R_{c}, and the carbon atom to which they are attached together comprise cyclopropyl or deuterated cyclopropyl;
R is hydrogen, 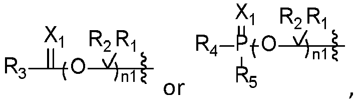 wherein:
   X₁ is an oxygen atom or a sulfur atom;
   n1 is 0, 1 or 2;
   each R₁ or R₂ is independently selected from the group consisting of hydrogen, deuterium, methyl and deuterated methyl;
   R₃ is selected from the group consisting of C1-C8 alkyl, C1-C8 alkoxy, C1-C8 alkylthio and C1-C8 alkylamino, wherein one or more hydrogen atoms of the said groups are substituted or unsubstituted by deuterium;
   R₄ and R₅ are each independently hydroxy; or are each independently selected from the group consisting of C1-C8 alkoxy, C1-C8 alkylthio, C1-C8 alkylamino, C6-C20 aralkyloxy, one or more hydrogen atoms of each of the said groups being substituted or unsubstituted by deuterium; and R₄ and R₅ together with the phosphorus atom to which they are attached form a 5-7 membered ring of 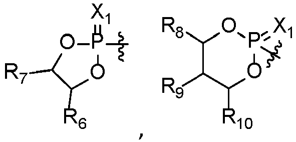 or 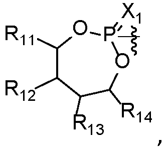 **wherein** R₆, R₇, R₈, R₉, R₁₀, R₁₁, R₁₂, R₁₃, and R₁₄ are each independently hydrogen or C1-C3 alkyl, or R₆ and R₇, R₈ and R₉, R₁₁ and R₁₂, R₁₂ and R₁₃ each together with the carbon atoms to which they are attached form an aromatic ring, wherein one or more of the hydrogen atoms in the substituent of the 5-7 membered ring formed by R₄ and R₅ together with the phosphorus atom to which they are attached can be substituted or unsubstituted by deuterium.

In some embodiments, the present application provides a compound represented by formula (II) or its hydrate, solvate, optical isomer, polymorph, isotopic derivative, or pharmaceutically acceptable salt, wherein: 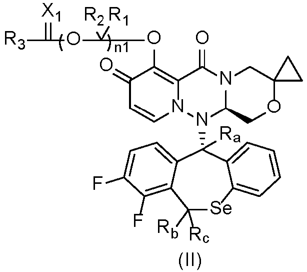 the definitions of the substituents in Formula (II) are as defined in Formula (I-0) and/or Formula (I).

In some embodiments, the present application provides a compound, hydrates, solvates, optical isomers, polymorphs, isotopic derivatives, or pharmaceutically acceptable salts thereof, as shown in Formula (III), wherein: 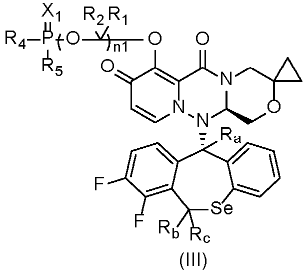 the definitions of the substituents in Formula (III) are as defined in Formula (I-0) and/or Formula (I).

In embodiments of the present application, the solvate refers to a complex formed by the interaction of a compound with a pharmaceutically acceptable solvent, including ethanol, isopropanol, acetic acid, ethanolamine.

In embodiments of the present application, the C1-C8 alkyl refers to a straight chain or branched chain saturated aliphatic hydrocarbon group having 1 to 8 carbon atoms in the molecule, including but not limited to methyl, ethyl, propyl, isopropyl, n-butyl, isobutyl, tert-butyl, n-pentyl, and the like.

In embodiments of the present application, the C1-C8 alkoxy, C1-C8 alkylthio refers to a group in which a saturated aliphatic hydrocarbon group having 1 to 8 carbon atoms in the molecule is inserted with an oxygen atom or a sulfur atom at any reasonable position, including but not limited to methoxy, ethoxy, propoxy, isopropoxy, isobutoxy, 2-ethyl ethoxy, methylthio, ethylthio, propylthio, isopropylthio, isobutylthio, and the like.

In embodiments of the present application, the C1-C8 alkylamino group refers to a group in which a saturated aliphatic hydrocarbon group having 1 to 8 carbon atoms is inserted with -NH- or -NH₂ group at any reasonable position in the molecule, including monoalkylamino, dialkylamino and cycloalkylamino groups, including but not limited to methylamino, ethylamino, propylamino, isopropylamino, dimethylamino, diethylamino, di-n-propylamino, diisopropylamino, and the like.

In embodiments of the present application, the C1-C3 alkyl group refers to an alkane having 1 to 3 carbon atoms in the molecule, including methyl, ethyl, propyl, isopropyl, cyclopropyl.

In embodiments of the present application, the C6-C20 aralkoxy group refers to a group in which an aralkyl group having 6 to 20 carbon atoms is linked to an oxygen atom, including but not limited to benzyloxy, phenethyloxy, naphthymethyloxy.

In embodiments of the present application, the deuterium substitution refers to the substitution of a hydrogen atom with a deuterium atom at any reasonable position in the molecule.

In some embodiments, Rₐ is hydrogen; in some embodiments, Rₐ is deuterium; in some embodiments, Rₐ is methyl; in some embodiments, Rₐ is deuterated methyl.

In some embodiments, R_{b} and R_{c} are both hydrogen; in some embodiments, R_{b} and R_{c} are both deuterium; in some embodiments, R_{b} is hydrogen and R_{c} is deuterium;
In some embodiments, R_{b} and R_{c} are each independently C1-C3 alkyl; in some embodiments, R_{b} is hydrogen and R_{c} is C1-C3 alkyl; in some embodiments, R_{b} is deuterium and R_{c} is C1-C3 alkyl;
In some embodiments, R_{b} and R_{c} are each independently deuterated C1-C3 alkyl; in some embodiments, R_{b} is hydrogen and R_{c} is deuterated C1-C3 alkyl; in some embodiments, R_{b} is deuterium and R_{c} is deuterated C1-C3 alkyl;
In some embodiments, R_{b} is C1-C3 alkyl and R_{c} is deuterated C1-C3 alkyl;
In some embodiments, R_{b}, R_{c}, and the carbon atom to which they are attached, together form cyclopropyl; in some embodiments, R_{b}, R_{c}, and the carbon atom to which they are attached, together form deuterated cyclopropyl.

In some embodiments, X is S. Furthermore, Formula (I-0) contains at least one deuterium atom.

In some embodiments, X is Se.

In some embodiments, R is hydrogen; in some embodiments, R is 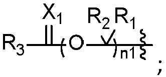 in some embodiments, R is

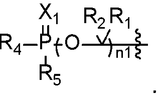

In some embodiments, n1 is 0; in some embodiments, n1 is 1; in some embodiments, n1 is 2.

In some embodiments, X₁ is an oxygen atom; in some embodiments, X₁ is an sulfur atom.

In some embodiments, R₁ and R₂ are both hydrogen; in some specific embodiments, R₁ or R₂ are both deuterium; in some embodiments, R₁ and R₂ are both methyl; in some embodiments, R₁ and R₂ are both deuterated methyl;
In some specific embodiments, R₁ is methyl and R₂ is hydrogen; in some specific embodiments, R1 is deuterated methyl and R₂ is hydrogen;
In some specific embodiments, R₁ is deuterated methyl and R₂ is deuterium.

In some embodiments, R₃ is selected from the group consisting of C1-C8 alkyl, C1-C8 alkoxy, C1-C8 alkylthio and C1-C18 alkylamine; preferably, R₃ is selected from the group consisting of C1-C8 alkyl and C1-C8 alkoxy; more preferably, R₃ is C1-C8 alkoxy;
In some specific embodiments, R₃ is selected from the group consisting of C1-C8 alkyl, C1-C8 alkoxy, C1-C8 alkylthio and C1-C18 alkylamine wherein one or more hydrogen atoms are substituted by deuterium; preferably, R₃ is selected from the group consisting of C1-C8 alkyl and C1-C8 alkoxy wherein one or more hydrogen atoms are substituted by deuterium; more preferably, R₃ is C1-C8 alkoxy wherein one or more hydrogen atoms are substituted by deuterium.

In some specific embodiments, n1 is 1, X₁ is an oxygen atom, R₁ and R₂ are both hydrogen, and R₃ is C1-C8 alkoxy or C1-C8 alkyl;
In some specific embodiments, n1 is 1, X₁ is an oxygen atom, R₁ and R₂ are both hydrogen, and R₃ is C1-C8 alkoxy wherein one or more hydrogen atoms are substituted by deuterium, or C1-C8 alkyl wherein one or more hydrogen atoms are substituted by deuterium.

In some specific embodiments, n1 is 0, X₁ is an oxygen atom, and R₃ is C1-C8 alkoxy or C1-C8 alkyl.

In some specific embodiments, n1 is 0, X₁ is an oxygen atom, and R₃ is a C1-C8 alkoxy group in which one or more hydrogen atoms are replaced with deuterium, or a C1-C8 alkyl group in which one or more hydrogen atoms are replaced with deuterium.

In some embodiments, R₄ and R₅ are each a hydroxyl group; in some embodiments, R₄ and R₅ are a C1-C8 alkoxy group, a C1-C8 alkylthio group, a C1-C8 alkylamino group; in some embodiments, R₄ and R₅ are a C6-C20 aralkyloxy group, preferably, R₄ and R₅ are a benzyloxy group;
In some embodiments, R₄ and R₅ are a C1-C8 alkoxy group, a C1-C8 alkylthio group or a C1-C8 alkylamino group, wherein one or more hydrogen atoms of each of the said groups being substituted or unsubstituted by deuterium;
In some embodiments, R₄ and R₅ together with the phosphorus atom to which they are attached form a 5-7 membered ring of 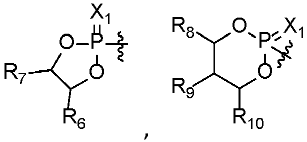 or 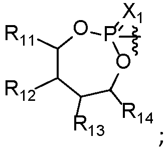 **wherein** R₆, R₇, R₈, R₉, R₁₀, R₁₁, R₁₂, R₁₃, and R₁₄ are each independently hydrogen or C1-C3alkyl, or R₆ and R₇, R₅ and R₉, R₁₁ and R₁₂, R₁₂ and R₁₃ each together with the carbon atoms to which they are attached form an aromatic ring,

In some embodiments, R₄ and R₅ together with the phosphorus atom to which they are attached form a 5-7 membered ring of 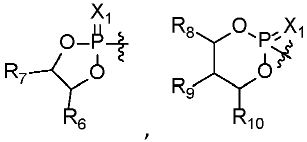 or 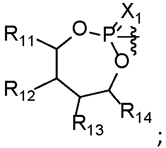 wherein one or more of the hydrogen atoms in the substituent of the 5-7 membered ring formed by R₄ and R₅ together with the phosphorus atom to which they are attached can be substituted or unsubstituted by deuterium.

In some specific embodiments, n1 is 1, X₁ is an oxygen atom, R₁ and R₂ are each hydrogen, and R₄ and R₅ are each a hydroxyl group; in some specific embodiments, n1 is 0, X₁ is an oxygen atom, and R₄ and R₅ are each a hydroxyl group.

In some embodiments, when R is hydrogen, the pharmaceutically acceptable salt of the compound can be an alkali metal salt, an alkaline earth metal salt, an amine salt, and an amino acid salt; preferably, the pharmaceutically acceptable salt includes sodium salt, potassium salt, magnesium salt, zinc salt, amine salt, basic amino acid salt, etc.

In some embodiments, when R₄ and R₅ are each a hydroxyl group, the pharmaceutically acceptable salt of the compound can be an alkali metal salt, an alkaline earth metal salt, an amine salt, and an amino acid salt; preferably, the pharmaceutically acceptable salt includes sodium salt, potassium salt, magnesium salt, zinc salt, amine salt, basic amino acid salt, etc.

In an embodiment of the present application, the pharmaceutically acceptable salt is obtained by a conventional salt formation method.

In an embodiment of the present application, the pharmaceutically acceptable salt is confirmed by means of NMR, mass spectrometry, atomic absorption spectrometry, elemental analysis, melting point detection, and the like.

In an embodiment of the present application, formula (I-0) or/and formula (I) comprises two chiral centers, and the compound of the present application or its intermediate can be obtained as a single configuration compound by chiral separation.

In some specific embodiments, the compound of the present application is an optical isomer of a single configuration as represented in formula (I-0) or/and formula (I).

In an embodiment of the present application, the absolute configuration of the optical isomer of a single configuration is determined by electronic circular dichroism.

In an embodiment of the present application, the optical rotation of the racemate and the optical isomer of a single configuration is tested according to the method for optical rotation measurement in Chinese Pharmacopoeia 2020 edition-Volume IV-0621.

The comparative compounds synthesized according to the synthetic methods in the literature, patents and examples of the present application were used for the relevant biological tests after detection of their purity > 98%.

The compounds provided by the present application include, but are not limited to, the following compounds: 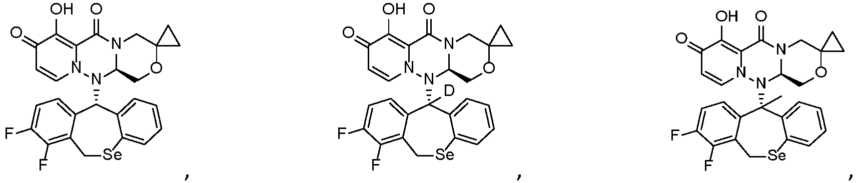 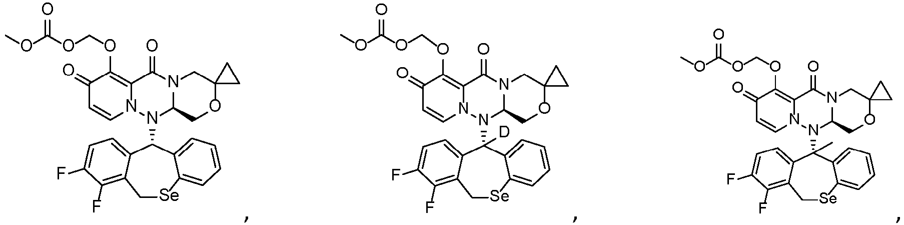 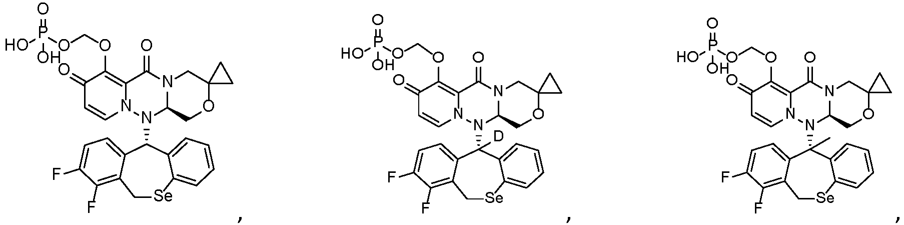 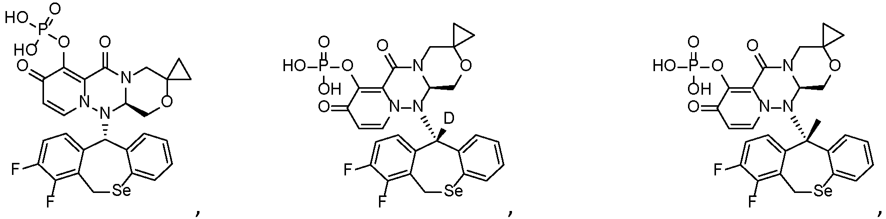 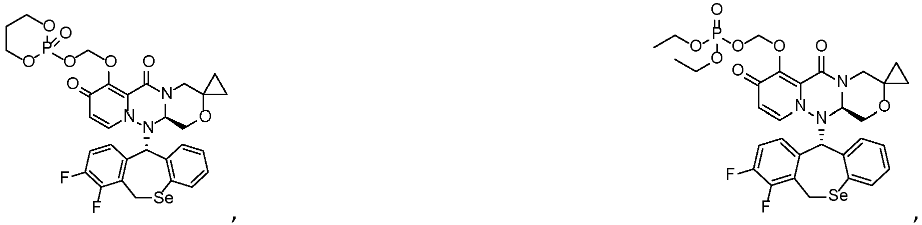 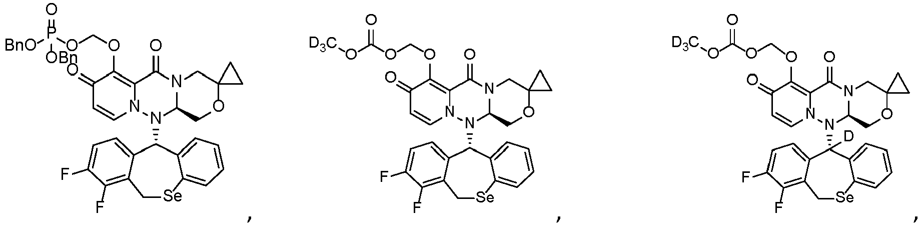 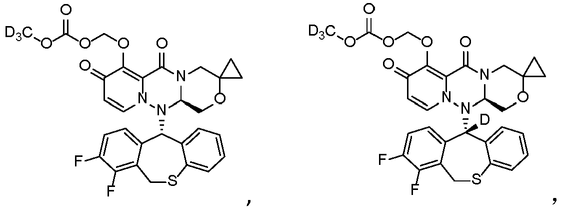 or their hydrate, solvate, optical isomer, polymorph, isotopic derivative, or pharmaceutically acceptable salt thereof.

The metal salts of the compounds provided by the present application include, but are not limited to, the following compounds:

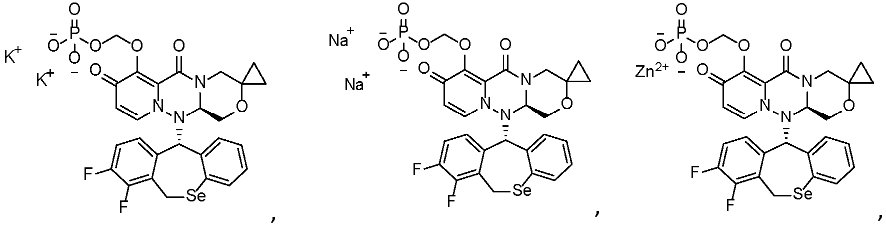

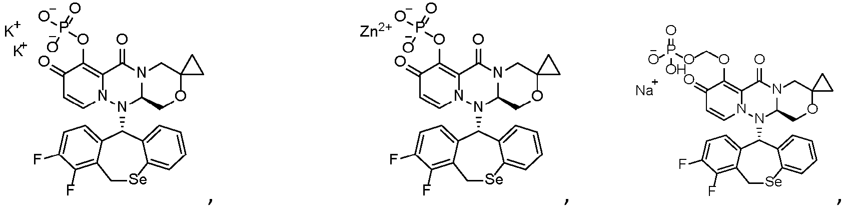

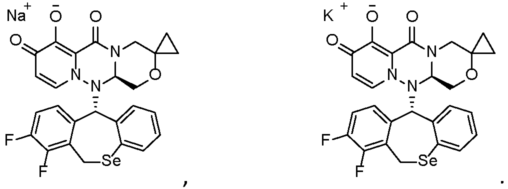

Another aspect of the present application provides pharmaceutical compositions comprising the above-mentioned compound or its hydrate, solvate, optical isomer, polymorph, isotopic derivative, or a pharmaceutically acceptable salt thereof, together with a pharmaceutically acceptable carrier; the pharmaceutically acceptable carrier includes one or a combination of a filler, a binder, a diluent, a lubricant, a preservative, a taste masking agent, or a solubilizer. The pharmaceutical composition can be used for anti-influenza virus.

Further, the dosage form of the pharmaceutical composition is tablet, capsule, powder, granule, pill, suspension, syrup, injection or inhalation preparation.

In a third aspect of the present application, the present application provides the above-mentioned compound, including its hydrates, solvates, optical isomers, polymorphs, isotopic derivatives, pharmaceutically acceptable salts thereof, or a pharmaceutical composition thereof, for use in anti-influenza virus.

The present application provides the use of the above-mentioned compound, including its hydrates, solvates, optical isomers, polymorphs, isotopic derivatives, pharmaceutically acceptable salts thereof, or a pharmaceutical composition thereof, in the preparation of a medicament for anti-influenza virus.

The present application provides a method for preventing or treating influenza virus infection, comprising administering to a patient in need of treatment a therapeutically effective amount of the above-mentioned compound, including its hydrates, solvates, optical isomers, polymorphs, isotopic derivatives, pharmaceutically acceptable salts thereof.

The compound of the present application has stronger antiviral activity *in vivo* or *in vitro,* and shows a better safety range;
The compound of the present application is effective against various influenza virus strains, including avian influenza virus strains, and is also effective against oseltamivir-resistant influenza viruses and other resistant strains, and has better activity than other similar structures; in particular, it shows better activity in inhibiting influenza B;
The *in vivo* anti-influenza virus activity of the compound of the present application is significantly improved, with lower viral titers in the lungs, and histopathological results also show that the lung tissue lesions after treatment with the compound of the present application are milder;
Further studies have found that the compound of the present application has good lung tissue distribution in animal experiments, which is consistent with the results of lower viral titers in the lungs and milder lung tissue lesions in the in vivo anti-influenza virus experiments, and is more conducive to exerting its anti-influenza virus effect, and is expected to have great clinical value and therapeutic advantages.

Pharmacokinetic studies of the compound of the present application show that it has a smaller mean absorption time, indicating that it can be absorbed more quickly after oral administration, and thus exert its efficacy more quickly.

### BRIEF DESCRIPTION OF DRAWINGS

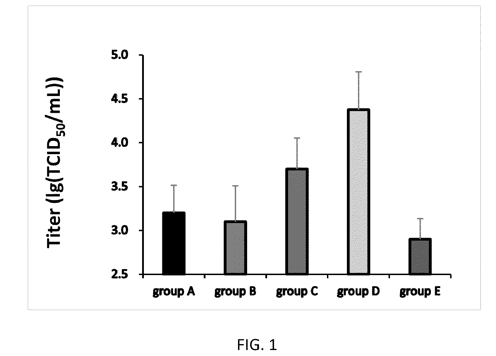

FIG. 1 is the lung tissue viral titers in the efficacy test of the influenza virus mouse model.

### DETAILED DESCRIPTION

The following examples can make the present application more comprehensible to those skilled in the art, but the present application is not limited thereto in any way, and the structures of all the compounds are confirmed by MS or ¹H-NMR, and the configuration of all the optical isomers involved is confirmed by optical rotation test or electronic circular dichroism.

In this example, unless otherwise specified, the solvents and reagents used are all common commercial products, and the starting materials are all commercially available raw materials.

### Embodiment 1: Synthesis of compound 9a

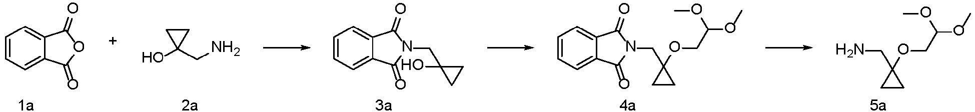

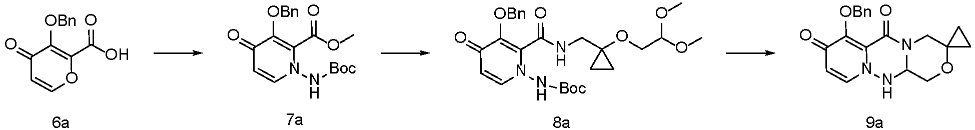

### Synthesis of compound 3a

5 g of compound 1a, 2.95 g of compound 2a and 7 ml of triethylamine were added to a mixed solvent of 40 ml of DMF (N,N-dimethylformamide) and 40 ml of toluene, the system was heated to 130°C for reaction for 8 hours. Water was added and stirred to precipitate solids. The system was filtered to obtain 7.03 g of white solid compound 3a. Without separation, it was directly used in the next reaction.

### Synthesis of compound 4a

5.84 g of compound 2-bromo-1,1-dimethoxyethane and 5 g of compound 3a were added to 40 ml of DCM, and 3.9 g of potassium tert-butoxide was slowly added to the system at room temperature. After addition, the system was heated to 40°C for reaction for 5 hours. The system was reduced to room temperature, water was added and stirred for 15 minutes, separated, the organic phase was concentrated to dryness, and purified by silica gel column to obtain 5.2 g of compound 4a, with a yield of 74%; ¹H NMR (CDCl₃,400MHz): δ7.83-7.88 (m, 2H ) , 7.68-7.70(m, 2H), 4.46(t,1H), 3.81(s,2H), 3.55(d,2H), 3.31(s, 6H), 0.47-0.95(m,4H).

### Synthesis of compound 5a

5 g of compound 4a were added to 15 ml of ethanol and 15 ml of water, and the system was heated to 60°C. 1.5 g of aqueous hydrazine hydrate solution (80%) was added, and the reaction was continued at 60°C for 5 hours. The system was concentrated to dryness, cooled to room temperature, and 40 ml of DCM and 40 ml of 1 N aqueous NaOH solution were added. The organic phase was separated, and the aqueous phase was further extracted twice with DCM, and the organic phases were combined. After the organic phase was dried over anhydrous sodium sulfate, it was concentrated to dryness to obtain 2.75 g of crude compound 5a, which was directly used in the next step without further separation.

### Synthesis of compound 7a

5g of compound 6a was added into 10ml of DMA, 1.62g of sodium hydride (60%) and 3.2g of methyl iodide were added. The system was stirred at 25°C for 12h, 100ml of water was added and stirred for 0.5h, extracted with EA. The organic phases were combined and washed with 0.5N dilute hydrochloric acid, saturated brine in turn, dried over anhydrous sodium sulfate and concentrated to dryness to give 4.65g of oil. 30ml of DMA, 3.54g of Boc hydrazine and 13.47g of pyridine p-toluene sulfonate were added to the oil. The system was heated to 60°C for 18h. After the reaction was completed, the system was extracted with water and EA, the organic phase was washed with saturated brine, dried over anhydrous sodium sulfate and concentrated to dryness. Purification on silica gel column gave 4.68g of compound 7a with a yield of 61.65%; the product was yellow oil, ESI-MS(+):m/z=375.2.

### Synthesis of compound 8a

4.5g of compound 7a was added into 30ml of ethanol, 24ml of 1N NaOH solution was added. The system was reacted at 60°C for 15h, the pH value was adjusted with dilute hydrochloric acid. The system was extracted with DCM (dichloromethane), the organic phases were combined and washed with saturated brine, dried over anhydrous sodium sulfate and concentrated to dryness to give off-white solid. The obtained off-white solid was added into DCM, 2.53g of compound 5a, 4ml of triethylamine and 6.85g of HATU were added at 25°C. The system was stirred at 25°C for 11h, diluted with water and extracted with DCM. The organic phases were combined and washed with saturated brine. After drying over anhydrous sodium sulfate, purification on silica gel column gave 4.1g of compound 8a with a yield of 66%; the product was white solid, ESI-MS(+):m/z=518.3.

### Synthesis of compound 9a

3g of compound 8a was added into 20ml of acetonitrile and 4ml of water, the system was heated to 60°C, 1.7g of methane sulfonic acid was added dropwise and the reaction was continued at this temperature for 5h. After the reaction was completed, the system was cooled to room temperature, aqueous sodium bicarbonate solution was added to adjust the pH to weak alkaline. The system was concentrated, extracted with DCM and water, the organic phase was separated, dried over anhydrous sodium sulfate and concentrated to dryness, purification on silica gel column gave 1.21g of compound 9a as white solid with a yield of 59%.ESI-MS(+):m/z=354.2.

### Embodiment 2: Synthesis of compound 21a

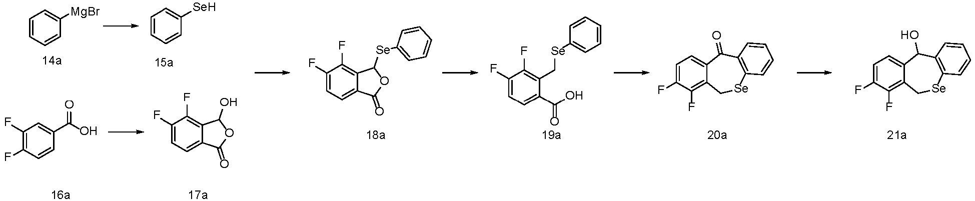

### Synthesis of compound 15a

After replacing the nitrogen in a three-necked flask, 226 ml of phenylmagnesium bromide (2.8 mol/L) was added, and the temperature was lowered to below 10°C in an ice water bath. Selenium powder 49.9 g was added in batches, and the reaction temperature was controlled to not exceed 30°C. After the addition of selenium powder was completed, the reaction was carried out for 2 hours. 2 mol/L hydrochloric acid was added in an ice water bath, and the reaction was exothermic. EA was added for extraction, and the organic phase was separated, dried and concentrated to obtain product 15a as a brown oil with a strong odor. The next step reaction was carried out directly.

### Synthesis of compound 17a

After replacing the nitrogen in a three-necked flask, 81.18 g of LDA (lithium diisopropylamide) was added, and the temperature was lowered to -30°C in a dry ice-ethanol solution. 50 g of a THF solution of compound 3,4-difluorobenzoic acid was added dropwise, and the reaction was exothermic during the dropwise addition. The temperature of the reaction system was controlled to be lower than -20°C, and the reaction was carried out for 2 hours. DMF was added, and the reaction was exothermic. The temperature of the reaction system was controlled to be lower than -20°C, and the reaction was gradually carried out at room temperature overnight. The reaction was determined to be completed by sampling. Hydrochloric acid was added to the reaction system, and the reaction was exothermic. EA was added for extraction, and the organic phase was separated, dried and concentrated to obtain 72 g of crude product 17a. The obtained product was directly used in the next step reaction. The obtained crude product was a yellow solid, and the yield was 122% (DMF was not completely removed by drying).

### Synthesis of compound 18a

After replacing the nitrogen in a three-necked flask, 58.81 g of compound 15a, 49.6 g of compound 17a, 300 ml of toluene and 17.6 g of camphorsulfonic acid were added, and the temperature was raised to 70°C. The reaction was carried out overnight. The reaction system was lowered to room temperature, and NaOH solution was added. The aqueous phase was extracted with EA, and the organic phases were combined and washed with saturated NaCl and then dried and concentrated. The crude product was 123.08 g. The obtained crude product was slurried with PE and then suction filtered to obtain 34.4 g of product. The slurry filtrate was recovered to obtain 14.83 g of product. Compound 18a was an orange solid, and the yield was 47.9%.

### Synthesis of compound 19a

Into a three-necked flask, 16.9 g of A1C1₃, 250 ml of toluene were added, the system was cooled in ice water bath, 17.1 g of tetramethyldisiloxane was added, stirred uniformly, 150 ml of toluene solution of compound 18a (34.4 g) was added, the reaction was slightly exothermic, A1C1₃ was dissolved gradually, heated to 80°C, reacted for 1 hour; stop the reaction, add sulfuric acid solution (16.2 mL + 240 mL water), separate the liquid, the aqueous phase was extracted with EA, the organic phase was spin-dried, the crude product was slurried with PE and then suction filtered to obtain 19a as yellow powdery solid 22 g, theoretical amount of product: 34.68 g, yield: 63.4%. Used directly in the next step reaction.

### Synthesis of compound 20a:

Into a three-necked flask, 429 g of polyphosphoric acid was added, heated to 80°C, 42 g of compound 19a was added, the temperature was raised to 120°C, the reaction system was viscous, the color changed from yellow to deep purple, reacted for 1 hour, sampled, added water, treated with EA; reduce the reaction temperature to below 100°C, add water and stir uniformly, extract with EA, separate the organic phase, spin dry, the crude product was slurried with PE and filtered to obtain 3.4 g of product, the filtrate was separated by column chromatography to obtain 10 g of product, compound 20a was colorless to pale yellow flocculent solid, theoretical yield: 39.69 g, actual yield:33.76%; ¹H NMR (DMSO-D6,400MHz): δ7.95-8.09(d.1H), 7.54-7.67(d,1H), 7.31-7.52(m,3H), 7.20-7.29(m,1H), 4.21-4.27(s,2H).

### Synthesis of compound 21a:

Into a three-necked flask, 2.12 g of compound 20a and 20 ml of ethanol were added, the system was stirred. Under ice water bath, sodium borohydride (0.26 g) solution in ethanol was added dropwise. The reaction system had a slight warming phenomenon, after the dropwise addition was completed. It was then transferred to room temperature reaction; the material was gradually dissolved. After the reaction solution was clear, sampled for detection. After the reaction is complete, 2 mol/L hydrochloric acid is added until no bubbles are generated. At PH4-6, a large amount of sold precipitate forms. The solid is then collected by suction filtration, the filtrate is extracted with EA, the organic phase was separated, the organic phase was spin-dried, and the solids were combined to obtain the product 21a as brown solid, which was used directly in the next step reaction.

### Embodiment 3: Synthesis of DS1491 and DS1494:

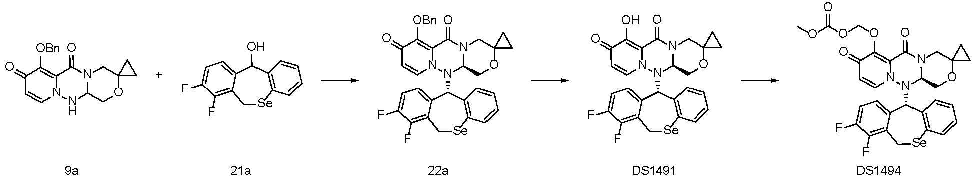

### Synthesis of compound 22a:

Into a flask, 0.8 g compound 9a, 0.77 g compound 21a, 1.8 g compound T3P (propylphosphonic anhydride, 50% EA solution), 0.33 g methane sulfonic acid and 10 ml of EA were added, heat to reflux, react overnight, take sample for detection, after the reaction is completed, carry out treatment; post treatment: add saturated sodium bicarbonate aqueous solution until no bubbles are released, separate, extract the aqueous phase with EA, combine the organic phases, spin dry, and purify through a silica gel column. Then prepare and separate through a chiral column to obtain 0.52 g compound 22a, yield: 37%.

### Synthesis of compound DS1491:

Into a flask, 0.5 g of compound 22a, 0.17 g of compound LiCl and 5 ml of DMA (N,N-dimethylacetamide) were added, warmed to 100°C, the reaction solution was yellow and turbid, reacted for 2 hours, sampled and detected, treated after the reaction was completed; saturated aqueous sodium bicarbonate solution was added, a solid precipitated, suction filtered, the filtrate was extracted with EA, the organic phase was spin dried, purified through a silica gel column to obtain 0.34 g of compound DS1491, yield 77%;ESI-MS(+):m/z=558.1.

### Synthesis of compound DS1494:

Into a flask, 0.3 g of compound DS1491, 150 mg of K₂CO₃, 10 mg of Kl and 110 mg of chloromethyl dimethyl carbonate were added. 5 mL of DMA was added to the system, warmed to 60°C, reacted overnight, sampled and detected, treated after the reaction was completed. The reaction system was cooled to room temperature, 2N HCl was added, water was added, a solid precipitated, EA and water were added for extraction, the organic phase was separated out, dried over anhydrous sodium sulfate, spin dried, separated by column chromatography to obtain 0.21 g of compound DS1494, yield: 61%. ESI-MS(+): m/z = 646.1.

### Embodiment 4: Synthesis of DS1492 and DS1495

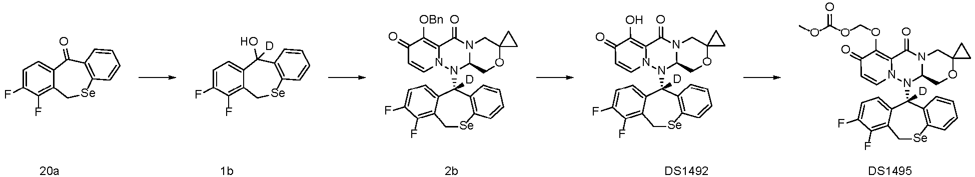

### Synthesis of compound 1b:

Compound 20a (1 g) was dissolved in THF (15 ml) under nitrogen protection, and lithium aluminum hydride-D4 (140 mg) was slowly added at 0°C. The system was warmed to 25°C and reacted for 8 hours. The system was cooled to 0°C, water was added to quench the reaction. The system was extracted with 2N hydrochloric acid and EA. The organic phase was concentrated to dryness, and column chromatography obtained compound 1b (0.58 g) with a yield of 57%, ESI-MS(+):m/z=314.0.

### Synthesis of compound 2b:

With reference to the synthesis method of compound 22a, compound 9a and compound 1b were used for synthesis to obtain compound 2b (0.36 g) with a yield of35%;ESI-MS(+):m/z=649.2.

### Synthesis of compound DS1492:

With reference to the synthesis method of compound DS1491, compound DS1492 was synthesized and obtained in a total amount of 0.21 g with a yield of 79%; ESI-MS(+):m/z=559.1.

### Synthesis of compound DS1495:

With reference to the synthesis method of compound DS1494, compound DS1495 was synthesized and obtained in a total amount of 0.13 g with a yield of 63%; ESI-MS(+):m/z=647.1.

### Embodiment 5: Synthesis of compounds DS1493 and DS1496

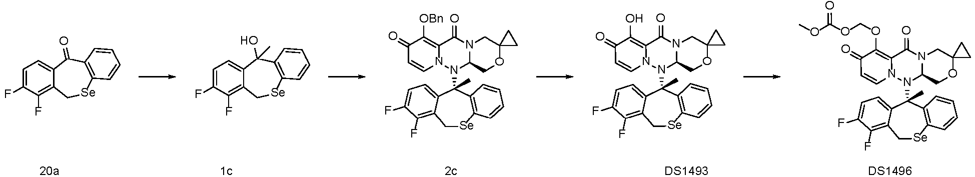

### Synthesis of compound 1c:

Under nitrogen, 1 g of compound 20a was dissolved in 15 ml of THF, and 4 ml of methyl lithium reagent (1.6 M in ether) was slowly added dropwise at -20°C, and the system was naturally warmed to 25°C for reaction for 8 hours. The system was cooled to 0°C, and water was added for quenching reaction. Concentration to dryness, extraction with EA and water. The organic phase was separated and concentrated to dryness. Column chromatography obtained 0.77 g of compound 1c in total, with yield of 73%, ESI-MS(+):m/z=327.1.

### Synthesis of compound 2c:

With reference to the synthesis method of compound 22a, compound 9a and compound 1c were used for synthesis, and 0.33 g of compound 2c was obtained in total, with yield of23%;ESI-MS(+):m/z=662.1.

### Synthesis of compound DS1493

With reference to the synthesis method of compound DS1491, 0.19 g of compound DS1493 was obtained in total, with yield of 73%; ESI-MS(+):m/z=571.1.

### Synthesis of compound DS1496

With reference to the synthesis method of compound DS1494, 95 mg of compound DS1496 was obtained in total, with yield of 55%; ESI-MS(+):m/z=660.1.

### Embodiment 6: Synthesis of compound DS1497

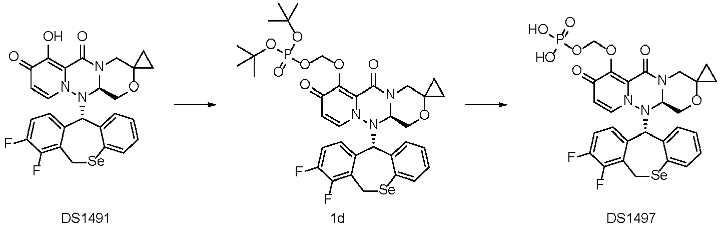

### Synthesis of compound 1d:

Into a flask, 1 g of compound DS1491, 0.7 g of tert-butyl chloromethyl phosphonate and 1 ml of triethylamine were added, and 10 ml of DMF (N,N-dimethylformamide) was added, and the system was reacted at 45°C for 8 hours. Water was added to the system to crystallize, and the compound 1d was obtained in a crude form of about 0.59 g with a yield of 42%, ESI-MS(+):m/z=779.5. The product was directly used in the next step without further purification.

### Synthesis of compound DS1497:

Under nitrogen protection, 0.5 g of compound 1d was added into a flask, followed by the addition of 5 ml of anhydrous dichloromethane. The mixture was stirred at room temperature, and 1 ml of trifluoroacetic acid was added dropwise. The mixture was stirred at 25°C for 3 hours. The system was concentrated to dryness, and isopropanol and water were added for crystallization to obtain 0.29 g of compound DS1497 in a yield of 69%, ESI-MS(+):m/z=668.1, ESI-MS(-):m/z=606.2.

### Embodiment 7: Synthesis of compound DS1831:

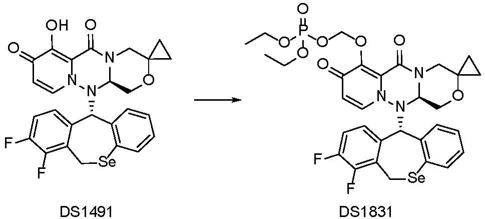

Into a single-necked flask, 70 mg of compound DS1491, 82 mg of Cs2CO3, 2.1 mg of Kl, 30 mg of compound chloromethyl diethyl phosphate and 10 mL of DMA were added. The system was heated to 50°C, and reacted for 2.5h, and the reaction was completed. The reaction system was cooled to room temperature, extracted with EA and water, and the organic phase was separated. The organic phase was dried over anhydrous sodium sulfate, rotary evaporated, and separated by column chromatography to obtain 55.45 mg of compound DS1831 in a yield of 61%, ESI-MS(+):m/z=724.1.

### Embodiment 8: Synthesis of compound DS1833:

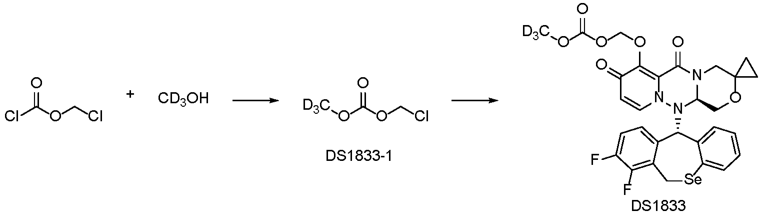

### Synthesis of compound DS1833-1

Into a flask, 1.1 g of deuterated methanol, 5.2 ml of triethylamine and 30 ml of dichloromethane were added, the system was cooled to 0°C, and 4 g of chloromethyl chloroformate was added dropwise slowly. After the dropping was completed, the system was naturally warmed to room temperature and reacted for 3 hours. The reaction was quenched by adding water, extracted by dichloromethane, and the organic phase was separated. The organic phase was dried over anhydrous sodium sulfate and concentrated to dryness to obtain 3.28 g of liquid compound DS1833-1, with a yield of 83%.

### Synthesis of compound DS1833

With compound DS1491 and DS1833-1 as materials, 78 mg of compound DS1833 was synthesized according to the synthesis method of compound DS1494, yield 55%, ESI-MS(+):m/z=649.1.

### Embodiment 9: Synthesis of compound DS14914:

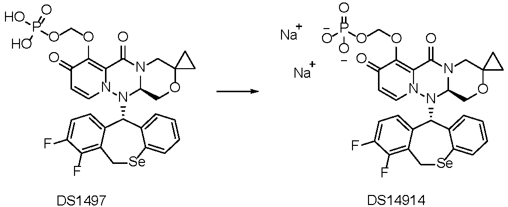

1g of compound DS1497 was added into 10ml of absolute ethanol, the system was warmed to 30°C, 120mg of sodium hydroxide was added, stirred for 2 hours, cooled to - 10°C, filtered, and the obtained product was dried under reduced pressure to obtain DS14914, with a yield of 90%, content of 99.7%, ESI-MS(+): m/z = 734.1 (M+Na), elemental analysis: found: Na, 6.33%; Se, 11.02%; theoretical value: Na, 6.47%; Se, 11.12%.

### Embodiment 10: Synthesis of compound DS14915:

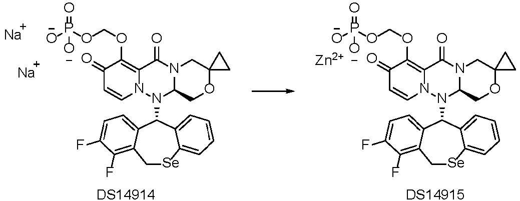

DS14914 (0.6 g) was dissolved in 25 ml deionized water, and a solution of zinc acetate dihydrate (185 mg) in deionized water (5 ml) was added. The mixture was stirred at room temperature for 2 hours, filtered, and the product was dried under reduced pressure to give DS14915 in 84% yield (99.4% purity), ESI-MS(+):m/z=729.9 (M+H) , Elemental analysis: Found: Se, 10.66%; Zn, 8.81%; Required: Se, 10.82%; Zn, 8.96%.

### Embodiment 11: Synthesis of compound DS1836:

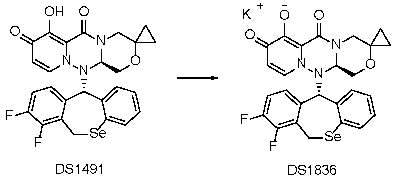

1g of compound DS1491 was added to 10ml of anhydrous ethanol, the system was warmed to 50°C, 100mg of potassium hydroxide was added, stirred for 2 hours, cooled to 0°C, filtered, and the obtained product was dried under reduced pressure to obtain DS1836, with a yield of 95%, and a content of 99.1%, ESI-MS(+):m/z=617.9 (M+Na), elemental Analysis: Found: K, 6.65%; Se, 13.15%; Theory: K, 6.58%; Se, 13.28%.

The following embodiment compounds were synthesized according to the same procedures as the above embodiment, using commercially available compounds or intermediate compounds synthesized from commercially available compounds:

| | | |
|---|---|---|
| 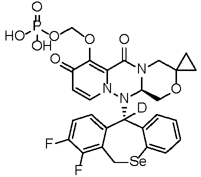 DS1498, | 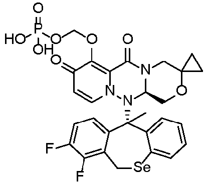 DS1499, | 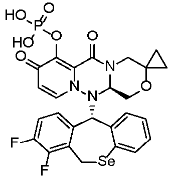 DS14910 |
| ESI-MS(+):m/z=669.1 | ESI-MS(+):m/z=682.1 | ESI-MS(-):m/z=636.0 |
| 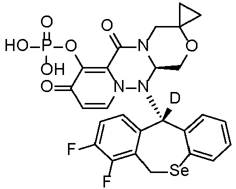 DS14911 | 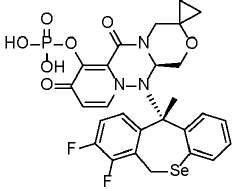 DS14912 | 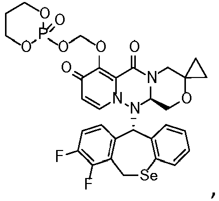 DS15812 |
| ESI-MS(-):m/z=636.9 | ESI-MS(-):m/z=650.1 | ESI-MS(+):m/z=708.1 |
| 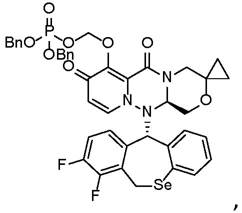 DS1832 | 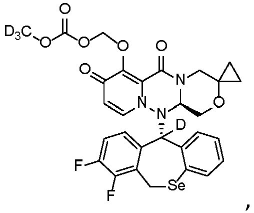 DS1834 | 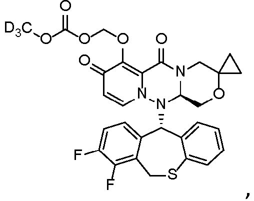 DS1837 |
| ESI-MS(+):m/z=848.2 | ESI-MS(+):m/z=650.2 | ESI-MS(+):m/z=601.2 |
| 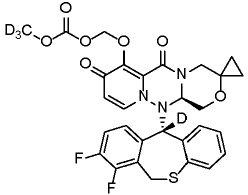 DS1838 | | |
| ESI-MS(+):m/z=602.2 | | |

Referring to the synthesis of metal salts in the present application and literature, the following embodiment compounds were obtained:

| | | |
|---|---|---|
| 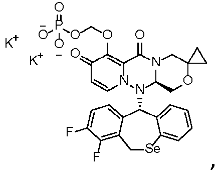 DS14913, ESI-MS(+):m/z=744.0 (M+H), elemental analysis :K, 10.44%;Se, 10.52% | 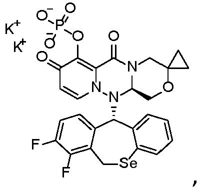 DS14916, ESI-MS(+):m/z=735.8 (M+Na), elemental analysis :K, 10.73%;Se, 10.95% | 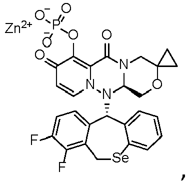 DS14917, ESI-MS(+):m/z=721.8 (M+Na), elemental analysis :Se, 11.15%; Zn, 9.44% |
| 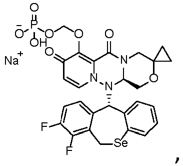 DS14918, ESI-MS(-):m/z=688.1 (M-H), elemental analysis :Na, 3.51%;Se, 11.38% | 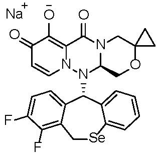 DS1835, ESI-MS(+):m/z=602.1 (M+Na), elemental analysis :Na, 4.13%;Se, 13.55% | |

### Comparative Example 1: Synthesis of compound M41 and M42

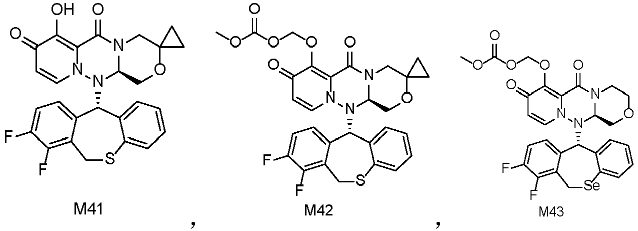

The two comparative compounds M41 and M42 were synthesized by referring to the synthetic methods of the reference compounds DS1491 and DS1494. Compound M43 was synthesized strictly in accordance with the method described in the literature (CN113226327B).

### Embodiment 12: Antiviral activity assay against influenza virus in cellular models

MDCK cells were inoculated into 96-well culture plates and cultured in a 5% CO2, 37°C incubator. When the cells were in the exponential growth phase, the cell culture maintenance solution containing different dilutions of the sample and the positive control drug was added, with three replicate wells for each concentration, and normal cell control wells were also set up. After 72 hours of culture after sample addition, the cytotoxicity test was performed using the CPE method. MDCK cells were also inoculated into 96-well culture plates and cultured in a 5% CO2, 37°C incubator. After 24 hours of culture, the cells were infected with influenza virus (A/HPB/359/95(H3N2)). After 2 hours of virus adsorption, the virus solution was discarded, and the cell culture maintenance solution containing different dilutions of the sample and the positive control drug was added, with three replicate wells for each concentration, and cell control wells and virus control wells were also set up. The plates were incubated in a 5% CO2, 37°C incubator. The antiviral test was performed using the CPE method, and the CPE was observed when the virus control group reached 4+ CPE. The TC50 (toxic concentration for 50% cells) and EC50 (effective concentration of drug for 50% cytopathic effect inhibition) of the sample were calculated using the Reed-Muench method, as shown in Table 1.

**Table 1: Cytotoxicity and influenza virus inhibition activity of compounds**

| Compounds | Anti-IAV EC₅₀ /nM | TC₅₀/µM in MDCK | Compounds | Anti-IAV EC₅₀ /nM | TC₅₀/µM in MDCK |
|---|---|---|---|---|---|
| DS1491 | 0.20±0.01 | >20 | DS1499 | 0.29±0.02 | >20 |
| DS1492 | 0.14±0.03 | >20 | DS14910 | 0.21±0.02 | >20 |
| DS1493 | 0.10±0.01 | >20 | DS14911 | 0.19±0.03 | >20 |
| DS1494 | 0.33±0.01 | >20 | DS14912 | 0.17±0.01 | >20 |
| DS1495 | 0.28±0.03 | >20 | M41 | 0.63±0.03 | >20 |
| DS1496 | 0.43±0.02 | >20 | M42 | 0.76±0.04 | >20 |
| DS1497 | 0.37±0.04 | >20 | Baricitinib ester | 0.68±0.06 | >20 |
| DS1498 | 0.31±0.02 | >20 | Baricitinib | 0.56±0.08 | >20 |
| DS15812 | 0.25±0.03 | >20 | DS1832 | 0.21±0.03 | >20 |
| 051831 | 0.19±0.02 | >20 | 051834 | 0.10±0.01 | >20 |
| DS1833 | 0.11±0.02 | >20 | 051838 | 0.52±0.04 | >20 |
| 051837 | 0.65±0.03 | >20 | | | |

| | | | | | |
|---|---|---|---|---|---|
| *Note: IAV in Table 1 represents A*/*Hangfang*/*359*/*95 (H3N2) strain* | | | | | |

Experiments show that the compounds of the present application are effective against in vitro anti-influenza virus activity and have large therapeutic index (TC50/EC50). Compared with the comparative compound M41 and baloxavir, the compound DS1491 and DS1493 (active ingredient) of the present application exhibit superior in vitro anti-influenza virus activity and a higher therapeutic index. Compared with the comparative compound M42 and baloxavir marboxil, the prodrug compounds of the present application also exhibit superior in vitro anti-influenza virus activity and a higher therapeutic index.

### Embodiment 13: Activity against other influenza viruses

The antiviral effects of the compounds on five real virus strains of influenza A virus (IAV) A/WSN/33 (H1N1), A/PR/8/34 (H1N1) (PR8), A/Weiss/43 (H1N1) oseltamivir-resistant strain, influenza B virus B/Beijing-Haidian/1386/2013 (BV), B/Massachusetts/2/2012 (BY-3) were determined according to the following method. Specifically, MDCK was inoculated in a 24-well plate at 1×105 cells per well, and after inoculation, it was placed in a 5% CO2, 37°C incubator for culture. After 24 h of cell culture, gradient-diluted test compound was added to each well. One hour after the addition of the compound, infection was performed with influenza viruses of different strains. Normal cell control wells and virus control wells were set. The antiviral test of the test sample was performed by the CPE method, and the degree of cytopathic effect (CPE) of each group was observed when the virus control group reached 4+ in degree of lesion (CPE). The EC50 of the sample was calculated by the Reed-Muench method, respectively. The experiment was repeated three times. The results are shown in Table 2.

**Table 2: Inhibitory activity against other influenza viruses**

| | EC₅₀ (nM) | | | | | | |
|---|---|---|---|---|---|---|---|
| Strains | DS1494 | DS14915 | DS1833 | 051837 | M42 | M43 | Ribavirin (µM) |
| A/WSN/3 3 | 1.16±0.2 6 | 1.13±0.3 1 | 1.02±0.2 7 | 1.36±0.2 2 | 2.28±0.4 2 | --- | 27.22±2.7 9 |
| PR8 | 1.05±0.1 9 | 0.97±0.3 6 | 0.89±0.2 2 | 1.26±0.4 7 | 1.93±0.5 5 | --- | 29.57±3.1 1 |
| OSE-R | 1.18±0.2 9 | 1.33±0.2 2 | 1.16±0.3 7 | 1.45±0.5 5 | 2.11±0.7 2 | --- | 30.33±4.0 8 |
| BY-3 | 2.90±0.3 4 | 2.18±0.5 6 | 2.77±0.7 5 | 5.88±0.9 1 | 7.24±1.3 6 | 11.32±2.5 3 | 33.14±3.2 9 |
| HD | 3.52±0.4 9 | 2.47±0.4 8 | 3.38±0.6 7 | 5.65±0.7 3 | 6.21±1.0 9 | 8.39±1.75 | 34.51±4.8 5 |

| | | | | | | | |
|---|---|---|---|---|---|---|---|
| *Note: A*/*WSN*/*33=A*/*WSN*/*33(H1N1); PR8=A*/*Puerto Rico*/*8*/*1934(H1N1) ;* *OSE-R= A*/*Weiss*/*43(H1N1)oseltamivir-resistant strains;* *HD=B*/*Beijing-Haidian*/*1386*/*2013 (BV); BY-3=BY*/*Massachusetts*/*02*/*2012* | | | | | | | |

Experiments show that the compounds of the present application have good activity against oseltamivir-resistant strains and influenza B virus. Compared with the compound M42 of the comparative example, the activity of the compounds in the present application is significantly improved, and is significantly higher than Ribavirin; the inhibitory activity of the compounds of the present application against influenza B virus is also significantly better than that of the compound M43 of the comparative example.

### Embodiment 14: Virus titer test and histopathological examination

Fifteen C57BL/6J mice (female, 8 weeks, 18-20g) were divided into 5 groups (group A, group B, group C, group D and group E), 3 in each group. After intranasal inoculation with a semi-lethal dose of A/PR/8/34 (H1N1) virus, group A/group B/group C were respectively given intragastric administration of test drugs (group A: DS1494; group B: DS1833; group C: M42, all suspended in 1% methylcellulose solution, and the dosage of each group was 2mg/kg), group D was given oral gavage with the same volume of 1% methylcellulose solution, and group E was given intraperitoneal injection of test drug DS1835 (the dosage was 1.4mg/kg, dissolved in physiological saline), and each group was given drug administration once a day. Lung tissue samples were collected 5 days after infection, the left lung was examined for pathology, and the right lung was homogenized for TCID50 determination. The results of virus titration are shown in Figure 1.

The results of lung virus titer test showed that: compared with the vehicle group, the lung tissue virus titers of other groups were lower; and compared with the compound M42 of the comparative example, the lung tissue virus titers of the mice in the DS1494 group and the DS1833 group of the present application were lower at the same dose, showing better anti-influenza virus effect; DS1835 was administered intraperitoneally at a relatively lower dose, and the results showed the lowest lung tissue virus titer.

The results of lung tissue pathology examination showed that: the lung tissue structure of the mice in group D (model) was obviously damaged, the alveolar structure was blurred, the local parenchyma was formed, and the epithelial cells were degenerated and necrotic; the lung tissue of the mice in group E (intraperitoneal injection of DS1835) had clear bronchial structure, and the epithelial cells had complete morphology and neat arrangement; the lung tissue of the mice in group A (intragastric administration of DS1494), group B (intragastric administration of DS1833) and group C (intragastric administration of M42) had clear bronchial structure, and the epithelial cells were arranged neatly with a small amount of inflammatory cell infiltration, and a small amount of epithelial cell necrosis was observed in the lung tissue of the mice in group C; compared with the compound M42 of the comparative example, the lung lesions of the mice treated with the compounds of the present application were milder, and the compounds of the present application could improve the pathological damage of lung tissue caused by influenza virus.

The above results show that the compounds of the present application have a better anti-influenza virus effect.

### Embodiment 15: Mouse Tissue Distribution Test

DBA/1J mice (6-8 weeks old, female) were weighed to calculate the dosing amount one day before administration. The mice were randomly divided into 2 groups (9 mice per group) and administered DS1494 and M42 (both 2 mg/kg, suspended in 1% methylcellulose solution) by oral gavage. The mice were sampled at the following 3 time points: 2 hours, 4 hours, and 8 hours (3 mice per time point). The mice were euthanized by CO2 at each time point. Organs (lungs, liver, kidneys, spleen, and heart) were collected from the euthanized mice, the blood was washed off the surface with normal saline, and the tissues were blotted dry with filter paper, weighed, placed in a glass homogenate tube, and homogenized with 3 times the volume of normal saline to obtain the respective tissue homogenates. The homogenates were stored at -80°C. The biological samples were processed and analyzed by LC-MS/MS (DS1491 was detected in the DS1494 group, and M41 was detected in the M42 group).

The results showed that: 2 hours after administration, both compounds reached the highest concentration in the above tissues, and in the lung tissue, the concentration of DS1491 in the DS1494 group was higher than that of M41 in the M42 group. And 4 hours and 8 hours after administration, the concentration of DS1491 in the DS1494 group decreased much more slowly than that of M41 in the M42 group; 8 hours after administration, the concentration of DS1491 in the DS1494 group was about 4 times that of M41 in the M42 group.

### Embodiment 16: Pharmacokinetic Study in Cynomolgus Monkeys

Cynomolgus monkeys (Macaca fascicularis), conventional grade, 24 individuals (half male and half female), aged 3-6 years, body weight 2.5-4 kg, were randomly divided into 8 groups labeled as Group A1, B1, C1, D1, and Group A2, B2, C2, D2. Groups A1, B1, C1, and D1 were administered orally via gavage (po) after an overnight fast with free access to water; Groups A2, B2, C2, and D2 were administered intravenously (iv) without prior fasting and with free access to water.

The samples were prepared into suspension with 0.5% CMC-Na aqueous solution for oral gavage administration, and the administration dose was 2 mg/kg; wherein M42 was given to group A1, DS1494 was given to group B1, DS1833 was given to group C1, and DS1837 was given to group D1. The samples were dissolved with mixed solvent (5% DMSO + 35% PEG400 + 60% water for injection) to prepare a solution for intravenous injection administration, and the administration dose was 0.5 mg/kg; wherein M42 was given to group A2, DS1494 was given to group B2, DS1833 was given to group C2, and DS1837 was given to group D2.

For the oral gavage group, blood was collected via femoral vein before administration (0h), 1 h, 2 h, 3 h, 4 h, 5 h, 6 h, 8 h, 12 h and 24 h after administration; for the intravenous injection group, blood was collected via femoral vein before administration (0h), 0.033 h, 0.083 h, 0.25 h, 0.5 h, 1 h, 2 h, 4 h and 24 h after administration. About 1.5 mL of blood collected each time was placed in a heparin anticoagulant tube, frozen and centrifuged at 2-8°C for 10 minutes, and LC/MS/MS was used to detect the target analyte in plasma (compound M41 was detected in M42 and DS1837 administration groups, and compound DS1491 was detected in DS1833 group and DS1494 group). The pharmacokinetic parameters were calculated by DAS based on the blood drug concentration data at different time points, and the results are shown in Table 3.

**Table 3: Pharmacokinetic Parameters in Cynomolgus Monkeys**

| Pharmacokinetic Parameters | unit of measurement | M42 | DS1494 | DS1833 | 051837 |
|---|---|---|---|---|---|
| Tmax* | h | 3 | 2 | 2 | 3 |
| MAT | h | 6.45 | 5.11 | 4.58 | 4.96 |

| | | | | | |
|---|---|---|---|---|---|
| ** is oral gavage data* | | | | | |

The results show that the time to reach peak Tmax and the mean absorption time MAT (MAT=MRT(po)-MRT(iv)) of the compounds according to the application are shorter, and that the compounds according to the application are absorbed rapidly after administration, compared to the reference compound M42. This characteristic is also shown by the other compounds according to the application.

The present application describes a plurality of embodiments, but the description is exemplary rather than limiting, and there can be more embodiments and implementation solutions within the scope of the embodiments described in the present application.

## Claims

1. A compound as shown in the following formula (I-0), or its hydrate, solvate, optical isomer, polymorph, isotopic derivative, or pharmaceutically acceptable salt thereof, wherein: 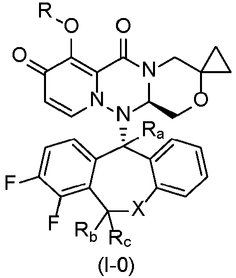 in the formula (I-0), Rₐ is selected from the group consisting of hydrogen, deuterium, methyl and deuterated methyl;
X is 5 or Se, and when X is S, at least one deuterium atom is contained in the formula (I-0);
R_{b} and R_{c} are each independently hydrogen, deuterium, C1-C3 alkyl, deuterated C1-C3 alkyl, or R_{b}, R_{c}, and the carbon atom to which they are attached together comprise cyclopropyl or deuterated cyclopropyl;
R is hydrogen, 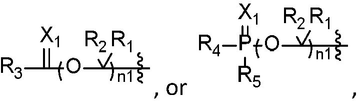 wherein
X₁ is an oxygen atom or a sulfur atom;
n1 is 0, 1 or 2;
each R₁ or R₂ is independently selected from the group consisting of hydrogen, deuterium, methyl and deuterated methyl;
R₃ is selected from the group consisting of C1-C8 alkyl, C1-C8 alkoxy, C1-C8 alkylthio and C1-C8 alkylamino, one or more hydrogen atoms of each of the said groups being substituted or unsubstituted by deuterium;
R₄ and R₅ are each independently hydroxy; or are each independently selected from the group consisting of C1-C8 alkoxy, C1-C8 alkylthio, C1-C8 alkylamino, C6-C20 aralkyloxy, one or more hydrogen atoms of each of the said groups being substituted or unsubstituted by deuterium; and R₄ and R₅ together with the phosphorus atom to which they are attached form a 5-7 membered ring of 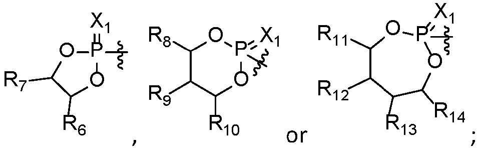 **wherein,** R₆, R₇, R₈, R₉, R₁₀, R₁₁, R₁₂, R₁₃ and R₁₄ are each independently hydrogen or C1-C3 alkyl, or R₆ and R₇, R₅ and R₉, R₁₁ and R₁₂, R₁₂ and R₁₃ each together with the carbon atom to which they are attached form an aromatic ring, wherein one or more of the hydrogen atoms in the substituent of the 5-7 membered ring formed by R4 and R5 together with the phosphorus atom to which they are attached can be substituted or unsubstituted by deuterium.

2. The compound according to claim 1, or its hydrate, solvate, optical isomer, polymorph, isotopic derivative, or pharmaceutically acceptable salt thereof, wherein: 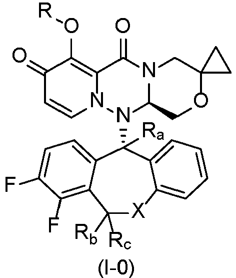 in Formula (I-0), Rₐ is selected from the group consisting of hydrogen, deuterium and methyl;
X is Se;
R_{b} and R_{c} are each independently hydrogen, deuterium, C1-C3 alkyl, deuterated C1-C3 alkyl, or R_{b}, R_{c}, and the carbon atom to which they are attached together comprise cyclopropyl or deuterated cyclopropyl;
R is hydrogen, 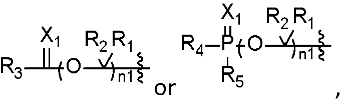 wherein:
X₁ is an oxygen atom or an sulfur atom;
n1 is 0, 1 or 2;
each R₁ or R₂ is independently selected from hydrogen and methyl;
R₃ is selected from the group consisting of C1-C8 alkyl, C1-C8 alkoxy, C1-C8 alkylthio and C1-C8 alkylamino;
R₄ and R₅ are each independently selected from the group consisting of hydroxy, C1-C8 alkoxy, C1-C8 alkylthio, and C1-C8 alkylamino.

3. A compound as represented by Formula (I), or its hydrate, solvate, optical isomer, polymorph, isotopic derivative, or pharmaceutically acceptable salt thereof, wherein: 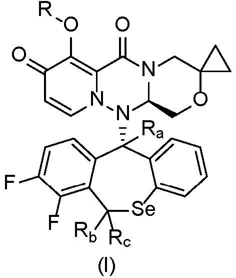 in Formula (I), Rₐ is selected from the group consisting of hydrogen, deuterium, methyl and deuterated methyl;
R_{b} and R_{c} are each independently hydrogen, deuterium, C1-C3 alkyl, deuterated C1-C3 alkyl, or R_{b}, R_{c}, and the carbon atom to which they are attached together comprise cyclopropyl or deuterated cyclopropyl;
R is hydrogen, 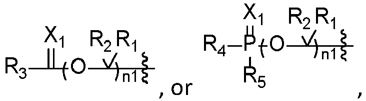 wherein
X₁ is an oxygen atom or a sulfur atom;
n1 is 0, 1 or 2;
each R₁ or R₂ is independently selected from the group consisting of hydrogen, deuterium, methyl and deuterated methyl;
R₃ is selected from the group consisting of C1-C8 alkyl, C1-C8 alkoxy, C1-C8 alkylthio and C1-C8 alkylamino, wherein one or more hydrogen atoms of the said groups are substituted or unsubstituted with deuterium;
R₄ and R₅ are each independently hydroxy; or are each independently selected from the group consisting of C1-C8 alkoxy, C1-C8 alkylthio, C1-C8 alkylamino, C6-C20 aralkyloxy, one or more hydrogen atoms of each of the said groups being substituted or unsubstituted by deuterium; and R₄ and R₅ together with the phosphorus atom to which they are attached form a 5-7 membered ring of 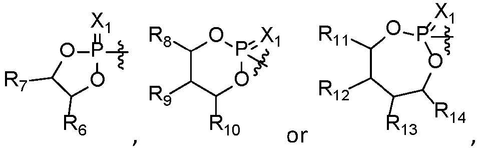 wherein R₆, R₇, R₈, R₉, R₁₀, R₁₁, R₁₂, R₁₃, and R₁₄ are each independently hydrogen or C1-C3 alkyl, or R₆ and R₇, R₅ and R₉, R₁₁ and R₁₂, R₁₂ and R₁₃ each together with the carbon atoms to which they are attached form an aromatic ring, wherein one or more of the hydrogen atoms in the substituent of the 5-7 membered ring formed by R₄ and R₅ together with the phosphorus atom to which they are attached can be substituted or unsubstituted by deuterium.

4. A compound as represented by Formula (II), or its hydrate, solvate, optical isomer, polymorph, isotopic derivative, or pharmaceutically acceptable salt thereof, wherein 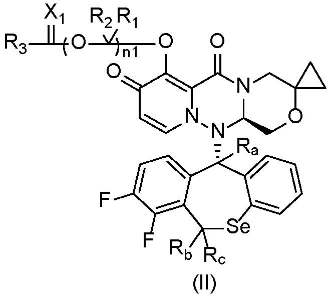 the definitions of the substituents in Formula (II) are as defined in claim 1.

5. A compound as represented by Formula (III), or its hydrate, solvate, optical isomer, polymorph, isotopic derivative, or pharmaceutically acceptable salt thereof, wherein: 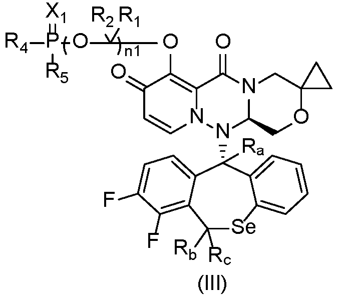 the definitions of the substituents in Formula (III) are as defined in claim 1.

6. The compound of any one of claims 1-5, selected from the structures: 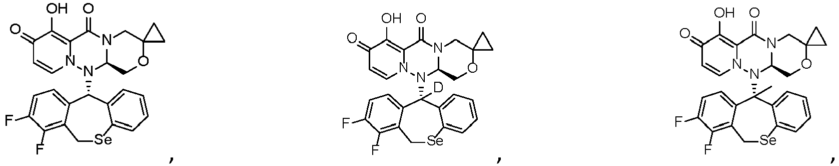 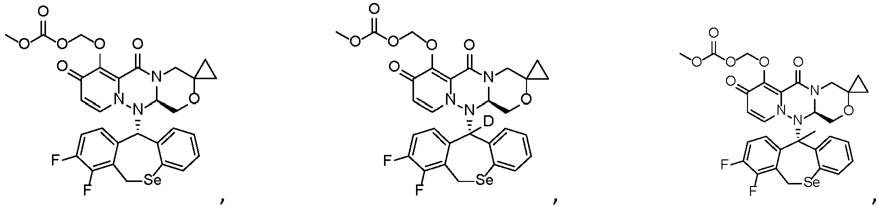 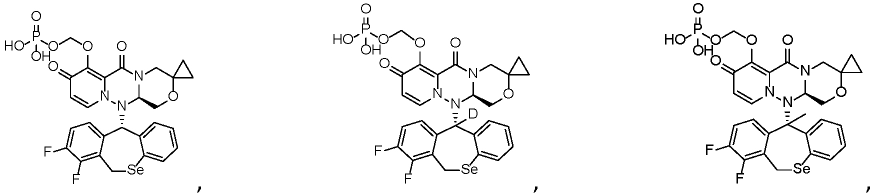 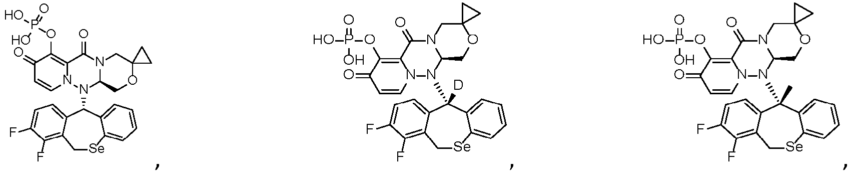 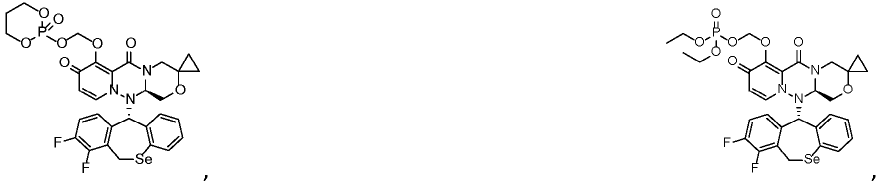 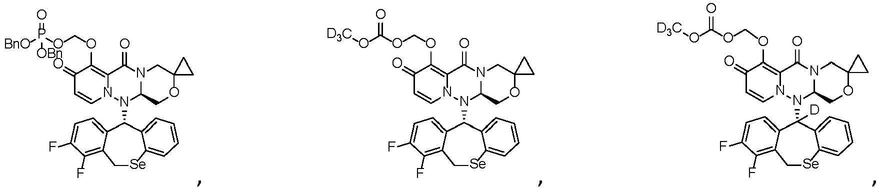 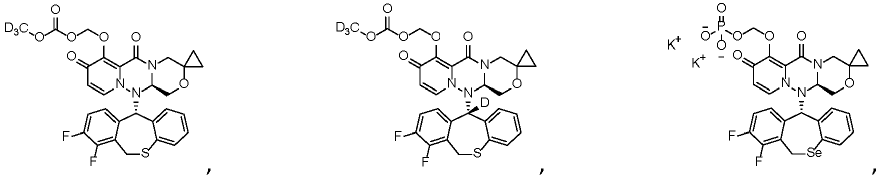 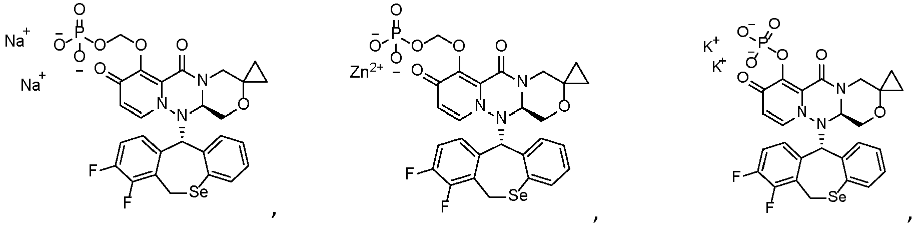 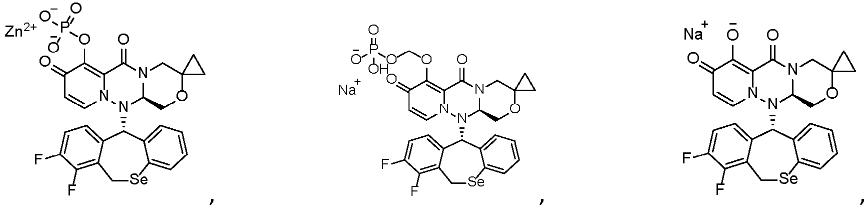 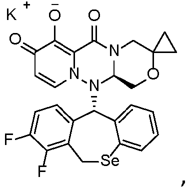 or its hydrate, solvate, optical isomer, polymorph, isotopic derivative, or pharmaceutically acceptable salt thereof.

7. A pharmaceutical composition comprising a compound of any one of claims 1-6, or its hydrate, solvate, optical isomer, polymorph, isotopic derivative, or pharmaceutically acceptable salt thereof, together with a pharmaceutically acceptable carrier.

8. The pharmaceutical composition of claim 7, in a dosage form of a tablet, capsule, powder, granule, pill, suspension, syrup, injection, or inhalation formulation.

9. The compound of any one of claims 1-6, or its hydrate, solvate, optical isomer, polymorph, isotopic derivative, pharmaceutically acceptable salt thereof, or the pharmaceutical composition of claim 7 or 8, for use against influenza virus.

10. A use of the compound of any one of claims 1-6, or its hydrate, solvate, optical isomer, polymorph, an isotopic derivative, a pharmaceutically acceptable salt thereof, or the pharmaceutical composition of claim 7 or 8, for the manufacture of a medicament against influenza virus.

11. A method of preventing or treating influenza virus infection comprising administering to a patient in need of treatment a therapeutically effective amount of the compound of any one of claims 1-6, or its hydrate, solvate, optical isomer, polymorph, isotopic derivative, pharmaceutically acceptable salt thereof, or the pharmaceutical composition of claim 7 or 8.

12. The compound of any one of claims 1-6, or its hydrate, solvate, optical isomer, polymorph, isotopic derivative, pharmaceutically acceptable salt thereof, or the pharmaceutical composition of claim 7 or 8, for use against influenza virus.
